# EUROPEAN PATENT APPLICATION

(11) **EP 4 835 817 A1**
(43) Date of publication of application: **04.11.2026**
(21) Application number: 26175361.0
(22) Date of filing: 29.04.2026
(51) Int. Cl.: G06T 7/60, G06T 7/00, G06T 7/62

(54) **ABNORMAL FEATURE ESTIMATION SYSTEM AND METHOD THEREOF**

(30) Priority: 30.04.2025 TW 114116430
(71) Applicant: ASUSTek Computer Inc., Peitou, Taipei-City 112 (TW)
(72) Inventor: WANG, Chia-Yu, 112 Taipei City (TW); CHEN, Yu-Hsin, 112 Taipei City (TW); SHEN, Yu-Jie, 112 Taipei City (TW)
(74) Representative: Viering, Jentschura & Partner mbB Patent- und Rechtsanwälte

(57) **Abstract**

An abnormal feature estimation system (10) and a method thereof are provided. An abnormality detection model (16) and an attribute feature estimation model (18) are implemented in a computing device (12). After receiving a real-time image (24), the abnormality detection model (16) detects an abnormal feature (26) of the real-time image (24), a selection box (28) is displayed around the abnormal feature (26), and position information is obtained. The attribute feature estimation model (18) calculates a lesion index (30) of the abnormal feature (26) based on the real-time image (24) and the position information. A display device (14) displays the real-time image (24) and the selection box (28). When the computing device (12) analyzes and determines that the real-time image (24) is a still image, the lesion index (30) is output, and the display device (14) displays the real-time image (24) and the lesion index (30). When the computing device (12) analyzes and determines that the real-time image (24) is a non-still image, the display device (14) stops displaying the lesion index (30).

## Description

### BACKGROUND OF THE INVENTION

### Field of the Invention

The present disclosure relates to an abnormal feature estimation system and an abnormal feature estimation method, which displays a lesion index in real-time when an image is analyzed and determined to be a still image.

### Description of the Related Art

An endoscope inspection instrument is used to examine organs or structures of a human body via an endoscope. The endoscope inspection instrument enters the human body through various body passages to observe internal conditions and determine whether there are any lesions. The endoscope inspection instrument comprises an esophageal endoscope, a gastroscope, or a colonoscope. Taking a colonoscope as an example, a flexible fiber endoscope is used to enter a colon for a direct visual inspection to confirm the presence of lesions such as polyps or tumorous changes. During the inspection, a colonoscope instrument is connected to a display to show real-time images of the internal intestinal structure captured by the instrument. Then, internal health status of the colon is examined or diagnosed by operators.

In the endoscopic examination process, a specific model button is configured for the endoscope to perform a dimension measurement function. When an operator detects a polyp and wants to estimate the polyp via the endoscope, the button needs to be pressed to capture an image and calculate the dimensions of the polyp. The polyp dimension information is then shown on the display. However, this examination method requires the endoscope with a specific button and specific operations, which is rather inconvenient and cannot be widely used in practice.

### BRIEF SUMMARY OF THE INVENTION

An abnormal feature estimation system, adapted to be electrically connected to an inspection instrument, the inspection instrument examines a target object and generates a real-time image. The abnormal feature estimation system comprises: a computing device, signal communicated to the inspection instrument, an abnormality detection model and an attribute feature estimation model are implemented in the computing device, the computing device receives the real-time image, the abnormality detection model detects an abnormal feature of the real-time image, a selection box is displayed around the abnormal feature, and position information is obtained, the attribute feature estimation model calculates a lesion index of the abnormal feature based on the real-time image and the position information, and the lesion index is output when the computing device determines the real-time image is a still image; and a display device, electrically connected to the computing device, the display device is configured to display the real-time image and the selection box, wherein when the computing device determines that the real-time image is the still image, the display device displays the real-time image and the lesion index, and when the computing device determines that the real-time image is a non-still image, the display device stops displaying the lesion index.

A method for abnormal feature estimation, adapted for a real-time image generated by an inspection instrument during examination of a target object, is also provided. The method comprises: receiving the real-time image; detecting an abnormal feature on the real-time image and obtaining position information; calculating a lesion index of the abnormal feature based on the real-time image and the position information; outputting the lesion index in response to activation of an image freeze function; displaying the real-time image and the lesion index; and not displaying the lesion index in response to deactivation of the image freeze function.

In summary, an abnormal feature estimation system and a method thereof capable of displaying the lesion index in real-time are provided. After the real-time image is obtained, an additional button is not required for the measurement function to display the lesion index. Instead, via the image freeze function of the inspection instrument, the lesion index is displayed on the display automatically when the system determines that the image is still. When the image freeze function is deactivated, the lesion index is not displayed. As a result, operators do not need to have too many operations on buttons during examination. Therefore, the present disclosure effectively assists operators by providing an accurate and easy-to-operate abnormal feature estimation system and method. Some functions are displayed in real-time by using existing capabilities of the inspection instrument, which provides intuitive and simple operations, thereby enhancing the user experience for operators.

### BRIEF DESCRIPTION OF THE DRAWINGS

FIG. 1 is a block diagram of an abnormal feature estimation system and an inspection instrument connected thereto in an embodiment;
FIG. 2 is a flowchart of an abnormal feature estimation method in an embodiment;
FIG. 3 is a schematic diagram of real-time images of an abnormal feature estimation system before, during, and after an image freeze function is activated in an embodiment.
FIG. 4 is a structural schematic diagram of the abnormal feature estimation system presenting a real-time image and a lesion index when an image freeze function is activated in an embodiment.

### DETAILED DESCRIPTION OF THE EMBODIMENTS

The embodiments of the present disclosure will be described below with reference to drawings. For clarity, some components or structures are omitted in the drawings of the embodiments to clearly show the technical features. In these drawings, the same reference numerals denote the same or similar components or circuits.

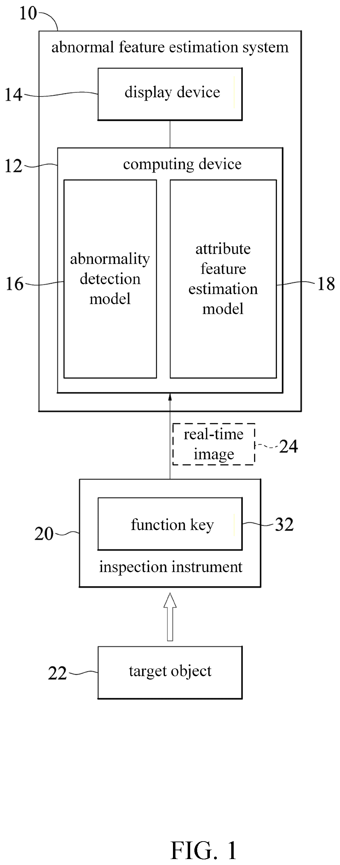

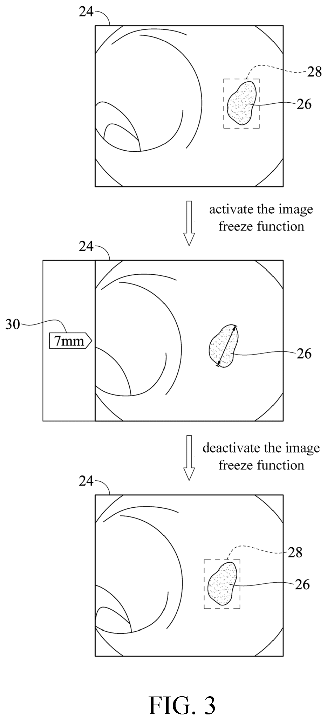

FIG. 1 is a block diagram of an abnormal feature estimation system and an inspection instrument connected thereto in an embodiment. Referring to FIG. 1, an abnormal feature estimation system 10 is connected to an inspection instrument 20. In one embodiment, the inspection instrument 20 examines a target object 22, captures images of the target object 22, and generates a real-time image 24 correspondingly. The real-time image 24 is transmitted to the abnormal feature estimation system 10. The abnormal feature estimation system 10 mainly comprises a computing device 12 and a display device 14. The computing device 12 is in signal connection with the inspection instrument 20. In one embodiment, the computing device 12 is electrically connected to the inspection instrument 20 via a Universal Serial Bus (USB) interface, a High-Definition Multimedia Interface (HDMI), or a Serial Digital Interface (SDI), which is not limited herein. The computing device 12 is electrically connected to the display device 14 via a High-Definition Multimedia Interface (HDMI), a DisplayPort (DP) interface, or a Serial Digital Interface (SDI), which is not limited herein. The computing device 12 comprises an abnormality detection model 16 and an attribute feature estimation model 18. An algorithm is implemented in the computing device 12. After the computing device 12 receives the real-time image 24 from the inspection instrument 20, the computing device 12 performs computational processing on the real-time image 24 to detect an abnormal feature 26 via the abnormality detection model 16. As shown in FIG. 3, the attribute feature estimation model 18 is used to calculate a lesion index 30 corresponding to the abnormal feature 26. When the computing device 12 analyzes and determines that the real-time image 24 is a still image through the algorithm, the lesion index 30 is output in real-time. The processed real-time image 24 from the computing device 12 is displayed at the display device 14. When the image freeze function is activated, the real-time image 24 and the lesion index 30 are displayed in real-time.

In one embodiment, the inspection instrument 20 is an endoscopic system, such as an esophageal endoscope, a gastroscope, or a colonoscope. When the inspection instrument 20 is an esophageal endoscope, the target object 22 is esophagus. When the inspection instrument 20 is a gastroscope, the target object 22 is cardia, pylorus, gastric fundus, or gastric body, etc. When the inspection instrument 20 is a colonoscope, the target object 22 is colon, cecum, ascending colon, transverse colon, descending colon, sigmoid colon, rectum, etc. In one embodiment, the abnormal feature comprises hyperplastic tissue or pathological tissue of the target object 22, such as polyps, tumors, or other formations on the target object tissue. In this embodiment, when the target object 22 is an intestinal tract, the abnormal feature is a colon polyp.

In one embodiment, the computing device 12, such as a computer or another electronic device with independent operation functions, cooperates with the display device 14, which is not limited herein. In another embodiment, a notebook computer is utilized to replace the functions of both the computing device 12 and the display device 14 to execute the tasks of the both.

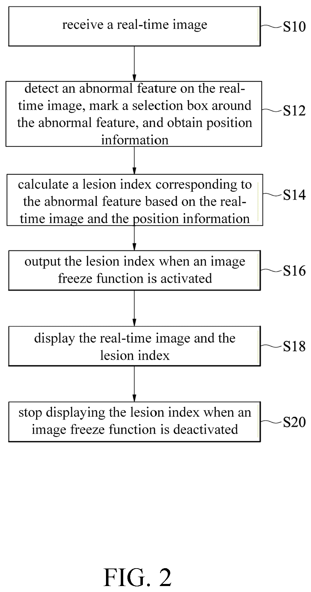

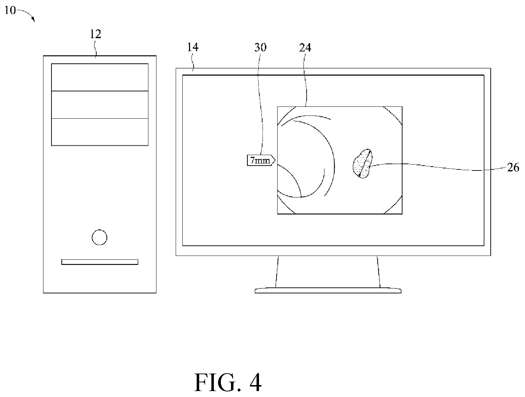

The steps of the abnormal feature estimation method executed by the abnormal feature estimation system 10 are described in detail with reference to FIG. 1. Please refer to FIG. 1, FIG. 2, and FIG. 3. After the inspection instrument 20 examines the target object 22 and generates the real-time image 24, as shown in step S10, the computing device 12 receives the real-time image 24 from the inspection instrument 20. At this time, the computing device 12 transmits the real-time image 24 to the display device 14, and then the real-time image 24 is displayed on the display device 14. As shown in step S12, the computing device 12 detects an abnormal feature 26 on the real-time image 24 via the abnormality detection model 16. As shown in FIG. 3, a selection box 28 is marked around the abnormal feature 26, and position information corresponding to this abnormal feature 26 is obtained. As shown in step S14, the computing device 12 executes the attribute feature estimation model 18. The attribute feature estimation model 18 calculates a lesion index 30 corresponding to the abnormal feature 26 based on the real-time image 24 and the position information. As shown in step S16, when the inspection instrument 20 is triggered to activate an image freeze function, the computing device 12 automatically analyzes whether the real-time image 24 is a still image via the algorithm. When the computing device 12 determines that the real-time image 24 is a still image, the computing device 12 outputs the lesion index 30 corresponding to the abnormal feature 26 based on the still image. As shown in step S18, the computing device 12 adds the lesion index 30 to the real-time image 24, and the real-time image 24 with the lesion index 30 is output at the display device 14, the display device 14 displays the real-time image 24 and the lesion index 30 in real-time. As shown in FIG. 3 and FIG. 4, in the embodiment, the lesion index 30 is a lesion size, which is not limited herein. Finally, as shown in step S20, when the inspection instrument 20 is triggered to deactivate the image freeze function, the computing device 12 automatically analyzes whether the real-time image 24 is a non-still image via the algorithm. When the computing device 12 determines that the real-time image 24 is the non-still image, the computing device 12 stops showing the lesion index 30 accordingly. The computing device 12 outputs the real-time image 24 and the selection box 28 to the display device 14 but not the lesion index 30.

In one embodiment, the attribute feature estimation model 18 is a size feature estimation model or a degree feature estimation model. When the attribute feature estimation model 18 is the size feature estimation model, the lesion index 30 is a lesion size of the abnormal feature 26. For example, the lesion size is the longest diameter of the esophageal cancer area, a polyp size (such as the polyp size of the colon, cecum, ascending colon, transverse colon, descending colon, sigmoid colon, rectum, cardia, pylorus, gastric fundus, or gastric body), or the ratio of maximum diameter of the intestinal metaplasia area. When the attribute feature estimation model 18 is the degree feature estimation model, the lesion index 30 is a lesion invasion degree or a lesion inflammation degree of the abnormal feature 26, such as the degree of esophageal cancer invasion or the degree of atrophic gastritis.

Please refer to FIG. 1, FIG. 3, and FIG. 4 simultaneously. In one embodiment the inspection instrument 20 is further provided with a function key 32, and the function key 32 is a built-in key on the inspection instrument 20 or a key on a keyboard (not shown) connected to the computing device 12. Taking the built-in key of the inspection instrument 20 as an example, the image freeze function of the inspection instrument 20 is activated via the function key 32, a still image is generated, and the display device 14 displays the real-time image 24 and the lesion index 30. Then, when the image freeze function is deactivated by pressing the function key 32 again, a non-still image is generated, and the display device 14 displays the real-time image 24 and the selection box 28 without showing the lesion index 30.

In one embodiment, regarding the algorithm implemented in the computing device 12, an additional hardware button is not necessary for the measurement function. The similarity between consecutive frames of the real-time image 24 is determined based on correlation and pixel-by-pixel comparison of the consecutive frames of the real-time image 24. Sampling is then applied for image comparison to meet the clinical requirements for the display of the real-time image 24 with the algorithm. Furthermore, the algorithm automatically analyzes the similarity between consecutive frames and quickly determines the changes of the frames. When the algorithm detects that the similarity between the frames meets a certain standard, a still image is determined, the lesion index is automatically displayed on the display. When a non-still image is determined, the lesion index is not displayed. As a result, operators do not need to have too many operations on buttons during examination.

In one embodiment, the abnormality detection model 16 and the attribute feature estimation model 18 (including the size feature estimation model and the degree feature estimation model) are a trained neural network model, respectively.

In summary, an abnormal feature estimation system and a method thereof capable of displaying a lesion index in real-time are provided. After a real-time image is obtained, an additional button is not required for the measurement function to display the lesion index. Instead, via an image freeze function of an inspection instrument, the lesion index is displayed on a display automatically when the system determines that the image is still. When the image freeze function is deactivated, the lesion index is not displayed. As a result, operators do not need to have too many operations on buttons during examination. Therefore, the present disclosure effectively assists operators by providing an accurate and easy-to-operate abnormal feature estimation system and method. Some functions are displayed in real-time by using existing capabilities of the inspection instrument, which provides intuitive and simple operations, thereby enhancing the user experience for operators.

The embodiments described above are intended merely to describe the technical concepts and features of the invention. The purpose is to enable those familiar with this technology to understand the content of the disclosure and implement it accordingly. This should not be used to limit the scope of the patent of the disclosure. In other words, any equivalent changes or modifications made based on the spirit disclosed in the disclosure should still be comprised in the scope of the patent of the disclosure.

## Claims

1. An abnormal feature estimation system (10), adapted to be electrically connected to an inspection instrument (20), the inspection instrument (20) examines a target object (22) and generates a real-time image (24), the abnormal feature estimation system (10) comprising:
a computing device (12), signal communicated to the inspection instrument (20), an abnormality detection model (16) and an attribute feature estimation model (18) are implemented in the computing device (12), the computing device (12) receives the real-time image (24), the abnormality detection model (16) detects an abnormal feature (26) of the real-time image (24), a selection box (28) is displayed around the abnormal feature (26), and position information is obtained, the attribute feature estimation model (18) calculates a lesion index (30) of the abnormal feature (26) based on the real-time image (24) and the position information, and the lesion index (30) is output when the computing device (12) determines the real-time image (24) is a still image; and
a display device (14), electrically connected to the computing device (12), the display device (14) is configured to display the real-time image (24) and the selection box (28), wherein when the computing device (12) determines that the real-time image (24) is the still image, the display device (14) displays the real-time image (24) and the lesion index (30), and when the computing device (12) determines that the real-time image (24) is a non-still image, the display device (14) stops displaying the lesion index (30).

2. The abnormal feature estimation system (10) according to claim 1, wherein the inspection instrument (20) is an endoscopic system.

3. The abnormal feature estimation system (10) according to claim 2, wherein the endoscopic system is an esophageal endoscope, a gastroscope, or a colonoscope.

4. The abnormal feature estimation system (10) according to claim 1, wherein the attribute feature estimation model (18) is a size feature estimation model or a degree feature estimation model.

5. The abnormal feature estimation system (10) according to claim 4, wherein when the attribute feature estimation model (18) is the size feature estimation model, the lesion index (30) is a lesion size of the abnormal feature (26).

6. The abnormal feature estimation system (10) according to claim 4, wherein when the attribute feature estimation model (18) is the degree feature estimation model, the lesion index (30) is a lesion invasion degree or a lesion inflammation degree of the abnormal feature (26).

7. The abnormal feature estimation system (10) according to claim 1, wherein the abnormal feature (26) comprises hyperplastic tissue or pathological tissue of the target object (22).

8. The abnormal feature estimation system (10) according to claim 1, wherein an algorithm is implemented in the computing device (12) for analyzing and determining whether the real-time image (24) is the still image or the non-still image.

9. The abnormal feature estimation system (10) according to claim 8, wherein the inspection instrument (20) further comprises a function key (32), when an image freeze function of the inspection instrument (20) is activated by pressing the function key (32), the display device (14) displays the real-time image (24) and the lesion index (30) when the computing device (12) analyzes and determines the real-time image (24) is the still image via the algorithm.

10. The abnormal feature estimation system (10) according to claim 9, wherein after the image freeze function is activated, when the image freeze function is deactivated by pressing the function key (32) again, the computing device (12) analyzes and determines that the real-time image (24) is the non-still image via the algorithm, and the display device (14) displays the real-time image (24) and the selection box (28) without showing the lesion index (30).

11. A method for abnormal feature estimation, adapted for a real-time image (24) generated by an inspection instrument (20) during examination of a target object (22), the method comprising:
receiving the real-time image (24);
detecting an abnormal feature (26) on the real-time image (24) and obtaining position information;
calculating a lesion index (30) of the abnormal feature (26) based on the real-time image (24) and the position information;
outputting the lesion index (30) in response to activation of an image freeze function;
displaying the real-time image (24) and the lesion index (30); and
stopping displaying the lesion index (30) in response to deactivation of the image freeze function.

12. The method for abnormal feature estimation according to claim 11, wherein the inspection instrument (20) is an endoscopic system.

13. The method for abnormal feature estimation according to claim 12, wherein the endoscopic system is an esophageal endoscope, a gastroscope, or a colonoscope.

14. The method for abnormal feature estimation according to claim 11, wherein the abnormal feature (26) is detected by an abnormality detection model (16), and a selection box (28) is marked around the abnormal feature (26).

15. The method for abnormal feature estimation according to claim 11, wherein the lesion index (30) is generated by an attribute feature estimation model (18).

16. The method for abnormal feature estimation according to claim 15, wherein the attribute feature estimation model (18) is a size feature estimation model or a degree feature estimation model.

17. The method for abnormal feature estimation according to claim 16, wherein when the attribute feature estimation model (18) is the size feature estimation model, the lesion index (30) is a lesion size of the abnormal feature (26).

18. The method for abnormal feature estimation according to claim 16, wherein when the attribute feature estimation model (18) is the degree feature estimation model, the lesion index (30) is a lesion invasion degree or a lesion inflammation degree of the abnormal feature (26).

19. The method for abnormal feature estimation according to claim 11, wherein the abnormal feature (26) is hyperplastic tissue or pathological tissue of the target object (22).

20. The method for abnormal feature estimation according to claim 11, wherein when the image freeze function is activated, an algorithm is used to analyze and determine whether the real-time image (24) is a still image or a non-still image.

21. The method for abnormal feature estimation according to claim 20, wherein the inspection instrument (20) further comprises a function key (32), when the image freeze function of the inspection instrument (20) is activated by pressing the function key (32), the display device (14) displays the real-time image (24) and the lesion index (30) when the computing device (12) analyzes and determines the real-time image (24) is the still image via the algorithm.

22. The method for abnormal feature estimation according to claim 21, wherein after the image freeze function is activated, when the image freeze function is deactivated by pressing the function key (32) again, the computing device (12) analyzes and determines that the real-time image (24) is the non-still image via the algorithm, and the display device (14) displays the real-time image (24) and the selection box (28) without showing the lesion index (30).
